# EUROPEAN PATENT APPLICATION

(11) **EP 4 224 161 A1**
(43) Date of publication of application: **09.08.2023**
(21) Application number: 21886429.6
(22) Date of filing: 01.11.2021
(51) Int. Cl.: G01N 33/48, G01N 33/493

(54) **REAGENT, KIT AND METHOD FOR STORING URINE SAMPLE**

(30) Priority: 02.11.2020 WO PCT/JP2020/041109
(71) Applicant: Healthcare Systems Co. Ltd., Nagoya-shi, Aichi 466-0058 (JP)
(72) Inventor: NAGAI Masashi, Nagoya-shi, Aichi 466-0058 (JP); ITO HAYASHI Michiko, Nagoya-shi, Aichi 466-0058 (JP)
(74) Representative: Mewburn Ellis LLP
(86) International application number: PCT/JP2021/040248
(87) International publication number: WO 2022/092312

(57) **Abstract**

Provided are a reagent, kit, and method for preserving a urine specimen used for analysis of urine components, enabling easy transportation of specimens and acquisition of analysis results equivalent to those of fresh urine even after transportation. A reagent for preserving a urine specimen, comprising a filter paper, wherein the filter paper comprises an aromatic alcohol, a phenol derivative, or a polyhydric fatty alcohol.

## Description

### Technical Field

The present invention relates to a reagent and kit for preserving a urine specimen used for tests for measuring urine components, and particularly to a reagent, kit, and method for suppressing concentration change of urine components during transportation.

### Background Art

Urine specimens of humans or animals comprise various biomarkers reflecting health conditions and presence or absence of diseases of subjects, i.e., the humans or animals, like blood samples. Unlike blood samples, urine specimens can be taken in a non-invasive manner. In the case the subject is a human, the subject can take urine specimens by himself. Because of this, over-the-counter test drugs (OTC test drugs) for urinalysis by subjects themselves at home, such as a urine sugar/urine protein test drug, a pregnancy test drug, and an ovulation test drug, have been widely spread. In addition to these OTC test drugs, urinalysis by mail is known. In urinalysis by mail, a subject takes a urine specimen and mails it to, e.g., a medical institution or a clinical laboratory, at which various components in urine are analyzed.

Urinalysis by mail is presumably carried out as follows: a subject puts a urine specimen directly in a container such as a polypropylene tube and mails it, or dips e.g., a filter paper in a urine specimen and dries it to prepare a urine-impregnated filter paper and then mails it. When a urine specimen directly put in a container is mailed, there is a risk of leakage due to cracks or defective sealing of a container. To reduce the risk, a urine-impregnated filter paper (for example, Non Patent Literature 1) may be used.

### Prior Art

### Non Patent Literature

Non Patent Literature 1: Koichi Takemori, Journal of the Japanese Society for Hygiene, Vol. 35, No. 5 (December 1980)

### Summary of Invention

### Object to be achieved

In urinalysis by mail, a risk such as leakage during transportation can be avoided by use of a urine-impregnated filter paper but it is questioned whether the same analysis results as in examinations using ordinary urine specimens can be obtained. Not only in urinalysis by mail but also in preserving/transporting urine, the levels of pH, salt concentration, and bacterial contamination of urine specimens vary from specimen to specimen, unlike blood. Depending on the environment during transportation, a urine specimen is oxidized, changes in properties, and decays. Thus, there is a risk that analysis results may differ from those obtained by fresh urine.

The present invention provides a reagent, kit, and method for preserving a urine specimen used for tests for measuring urine components, enabling easy transportation of specimens and acquisition of analysis results equivalent to those of fresh urine even after transportation.

### Means to Achieve Object

As a result of intensive studies, the present inventors found that the same analysis results as in fresh urine can be obtained by use of a urine-impregnated filter paper, which is prepared by impregnating a filter paper comprising an aromatic alcohol, a phenol derivative, or a polyhydric fatty alcohol with fresh urine, even after a time. Based on the finding, the present invention was accomplished. More specifically, the present invention provides the following:
(1) A reagent for preserving a urine specimen, comprising a filter paper, wherein the filter paper comprises an aromatic alcohol, a phenol derivative, or a polyhydric fatty alcohol.
(2) The reagent according to (1), wherein the content of the aromatic alcohol, phenol derivative, or polyhydric fatty alcohol in the filter paper is 0.5 to 100 mg per mL of a urine specimen to be applied.
(3) The reagent for preserving a urine specimen according to (1) or (2), wherein the filter paper comprises phenoxyethanol.
(4) The reagent according to (3), wherein the content of phenoxyethanol in the filter paper is 1.0 to 50 mg per mL of a urine specimen to be applied.
(5) The reagent according to any one of (1) to (4), wherein the urine specimen is a specimen for measuring a content of at least one selected from the group consisting of 8-hydroxydeoxyguanosine, vitamins, and metal ions.
(6) A kit comprising the reagent according to any one of (1) to (5).
(7) A method for preserving a urine specimen, comprising a step of contacting the urine specimen with a filter paper comprising an aromatic alcohol, a phenol derivative, or a polyhydric fatty alcohol.
(8) The method according to (7), wherein the content of the aromatic alcohol, phenol derivative, or polyhydric fatty alcohol in the filter paper is 0.5 to 100 mg per mL of the urine specimen to be applied.

### Advantageous Effects of Invention

According to the present invention, it is possible to provide a reagent, kit, and method for preserving a urine specimen used for tests for measuring urine components, enabling easy transportation of specimens and acquisition of analysis results equivalent to those of fresh urine even after transportation.

### Brief Description of Drawings

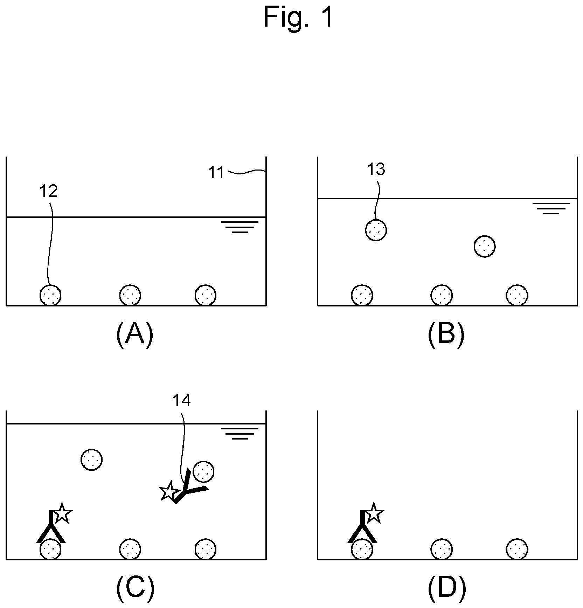

[Figure 1] Figure 1 is a schematic view showing an example of a measuring principle for 8-OHdG.

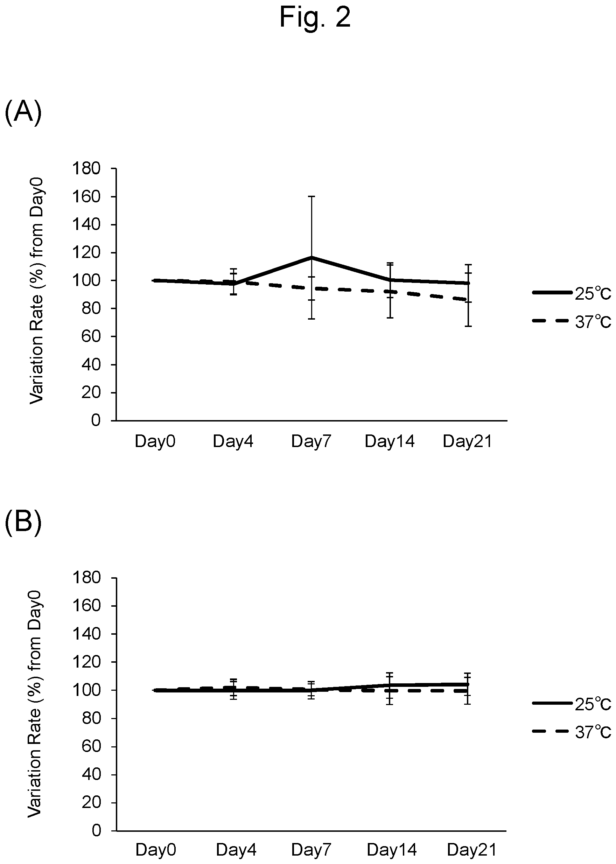

[Figure 2] Figure 2 discloses graphs showing the relationship between the variation rate of 8-OHdG measurement value and the incubation time. (A) shows the variation rate of the measurement value of urine-impregnated filter paper specimen not comprising phenoxyethanol and (B) shows the variation rate of the measurement value of the specimen comprising phenoxyethanol.

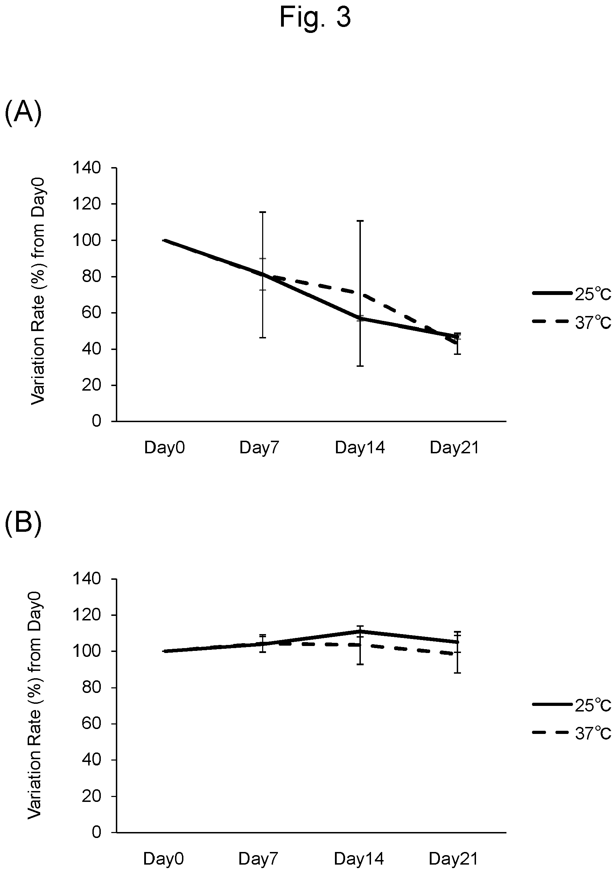

[Figure 3] Figure 3 discloses graphs showing the relationship between the variation rate of vitamin B1 measurement value and the incubation time. (A) shows the variation rate of the measurement value of urine-impregnated filter paper specimen not comprising phenoxyethanol and (B) shows the variation rate of the measurement value of the specimen comprising phenoxyethanol.

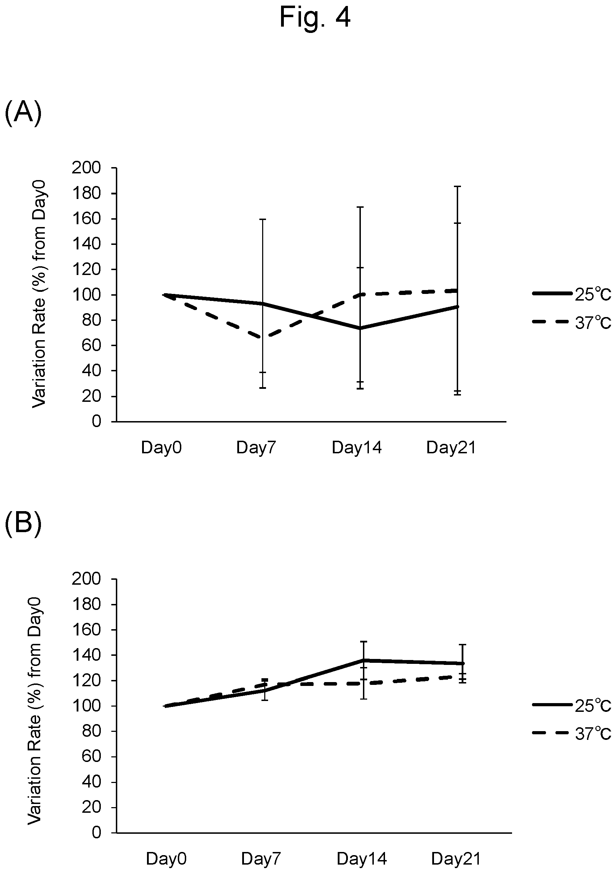

[Figure 4] Figure 4 discloses graphs showing the relationship between the variation rate of vitamin B2 measurement value and the incubation time. (A) shows the variation rate of the measurement value of urine-impregnated filter paper specimen not comprising phenoxyethanol and (B) shows the variation rate of the measurement value of the specimen comprising phenoxyethanol.

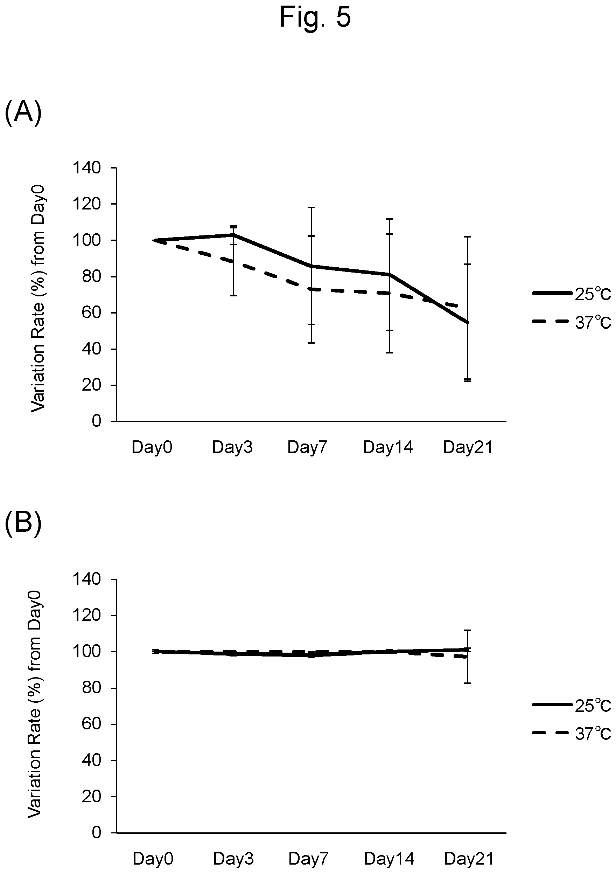

[Figure 5] Figure 5 discloses graphs showing the relationship between the variation rate of magnesium measurement value and the incubation time. (A) shows the variation rate of the measurement value of urine-impregnated filter paper specimen not comprising phenoxyethanol and (B) shows the variation rate of the measurement value of the specimen comprising phenoxyethanol.

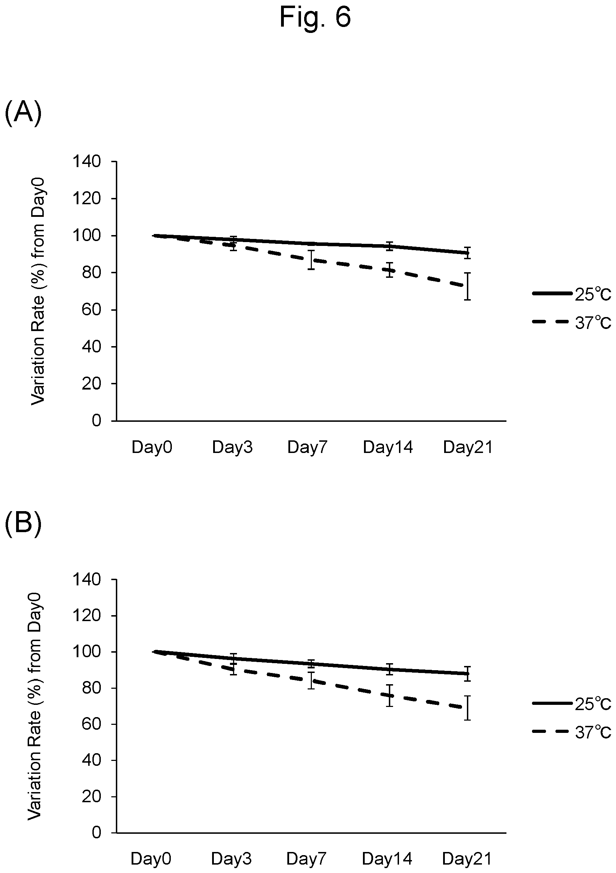

[Figure 6] Figure 6 discloses graphs showing the relationship between the variation rate of the creatinine measurement value in an ordinary specimen and the incubation time. (A) shows the variation rate of the measurement value of urine-impregnated filter paper specimen not comprising phenoxyethanol and (B) shows the variation rate of the measurement value of the specimen comprising phenoxyethanol.

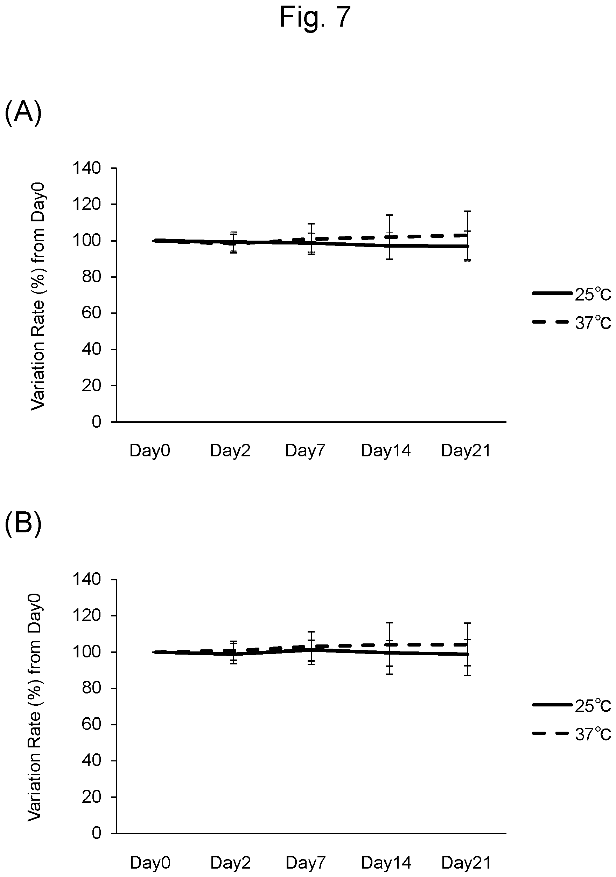

[Figure 7] Figure 7 discloses graphs showing the relationship between the variation rate of the creatinine measurement value in a dried specimen and the incubation time. (A) shows the variation rate of the measurement value of urine-impregnated filter paper specimen not comprising phenoxyethanol and (B) shows the variation rate of the measurement value of the specimen comprising phenoxyethanol.

### Description of Embodiments

In the specification, "A to B" (A and B represent numerical values) refers to "A or more and B or less" unless otherwise specified. In the specification, "%" refers to wt% unless otherwise specified.

### <1. Reagent>

The reagent is a reagent for preserving a urine specimen, comprising a filter paper, wherein the filter paper comprises an aromatic alcohol, a phenol derivative, or a polyhydric fatty alcohol. The "reagent" herein includes not only a compound but also a filter paper comprising the compound.

The "urine specimen" is an object to be preserved by the reagent of the present invention and taken from a subject such as a human or an animal whose urine is to be analyzed. The subject providing a specimen for urinalysis is not particularly limited as long as it is a human or an animal discharging urine. Examples of the subject include primates such as a human and a chimpanzee; pet animals such as a dog and a cat; livestock animals such as a cow, a pig, a horse, sheep, and a goat; rodents such as a mouse and a rat; and animals raised in zoos. A human is preferred.

Urine specimens have problems in that they significantly differ in, e.g., pH and salt concentration individually, and are likely to be contaminated with bacteria. Since urine specimens easily change in properties, urine components are usually measured, within a short period of time after urination, by using fresh urine or pooled urine within 24 hours. Alternatively, if a urine specimen has to be kept for a long period of time, the specimen is cryogenically preserved.

Assuming, e.g., urinalysis by mail requiring a subject to take own urine, it takes at least several days until measurement of urine components from urination. It is difficult to keep a urine specimen in a frozen state during the period, from the aspect of technique or cost. To prevent leakage due to cracks and defective sealing of a container, it is presumed to use a urine-impregnated filter paper prepared by impregnating a filter paper with urine. Also in this case, it is difficult to transport a urine-impregnated filter paper in a frozen state.

The present inventors checked a urine specimen, more specifically, a urine-impregnated filter paper allowed to stand still at room temperature for several days or more. As a result, they found that numerical values of some urinalysis items vary from those of fresh urine. More specifically, they found that, in a urine-impregnated filter paper allowed to stand still at room temperature, a measurement value of an oxidative stress marker, 8-hydroxydeoxyguanosine (8-OHdG) increases compared to that of fresh urine.

A urine specimen in the present invention refers to a urine-impregnated filter paper. The "urine-impregnated filter paper" refers to a filter paper strip impregnated with urine, which is prepared by bringing urine into contact with the filter paper strip (e.g., by dripping or dipping), and then drying the strip. The urine-impregnated filter paper used is defined as that prepared by drying a urine-impregnated filter paper within 7 days, preferably 3 days, and more preferably 1 day after taking a urine specimen until difference in weight of the filter paper from that before contact with urine becomes 10.0% or less, based on the weight difference between filter paper before and immediately after contact with urine regarded as 100%, in other words, the weight of the urine specimen becomes 10.0% or less of that immediately after contact with urine. Although a method for preparing such a urine-impregnated filter paper is not particularly limited, a urine-impregnated filter paper can be prepared, for example, by (1) bringing a filter paper strip into contact with urine, and thereafter, drying the filter paper strip in air at room temperature for several hours to overnight; (2) bringing a filter paper strip into contact with urine, and thereafter, putting the strip into a plastic bag with zipper comprising a desiccant such as silica gel and allowing it to leave at room temperature; or (3) bringing a filter paper strip into contact with urine, and thereafter, putting the strip in a plastic bag with zipper and allowing it to leave at room temperature. For the preparation methods (1) to (3), the time for drying the urine-impregnated filter paper usually gets shorter in the order of (2), (1), and (3).

A "filter paper" herein that can be used is the same as those used as a filter paper commonly used for a blood-impregnated filter paper. Although the material for the filter paper is not particularly limited, cellulose can be preferably used. Although the thickness, shape, and size thereof are not particularly limited, for example, a material having a thickness of 0.10 to 4.0 mm, a round, oval, square, or rectangular shape, and a major axis of 3.0 to 150 mm can be preferably used. The filter paper preferably has a water absorbing capacity of a predetermined vale or more. For example, a filter paper having a certain level of water absorbing capacity, e.g., a water absorbency (water absorption test according to JIS L 1907:2010 Byreck method) of 4.0 cm or more, particularly 6.0 cm, further 8.0 cm or more, can be preferably used.

The amount of a urine specimen to be used in the present invention is not particularly limited and preferably 20 to 500 µL, and particularly 50 to 200 µL in order to more easily dry a urine-impregnated filter paper. A urine-impregnated filter paper is preferably prepared by impregnating a filter paper with a predetermined volume of urine. Although a method for impregnating a filter paper with urine is not particularly limited, for example, a method of dripping urine on a filter paper by use of a dropper and a method of dipping a filter paper in urine in a urine cup for a predetermined time (for example, 3 seconds) can be used.

According to the reagent of the present invention, to prepare a urine-impregnated filter paper as mentioned above, it is necessary for a filter paper to comprose an aromatic alcohol, a phenol derivative, or a polyhydric fatty alcohol (hereinafter also referred to as "aromatic alcohol or the like"). The reagent of the present invention is prepared by impregnating a filter paper with a solution of an aromatic alcohol or the like and drying the filter paper. Although a method for impregnating a filter paper with a solution of an aromatic alcohol or the like is not particularly limited, any one of methods: dripping, spraying, and impregnating can be used. As a method for drying a filter paper impregnated with a solution, any methods can be used as long as it neither changes properties of an aromatic alcohol or the like nor reduces the content. For example, a method such as air dry and vacuum dry can be used.

The "aromatic alcohol" to be comprised in the reagent of the present invention is not particularly limited as long as it is an alcohol having an aromatic ring. An aromatic alcohol can be selected, for example, from benzyl alcohol, salicyl alcohol, phenethyl alcohol, phenoxyethanol, 3-phenyl-1-propanol, and derivatives thereof. Particularly, phenoxyethanol (Formula I) and phenethyl alcohol (Formula II) can be preferably used.

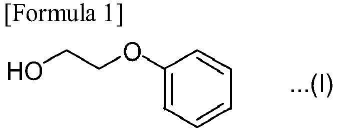

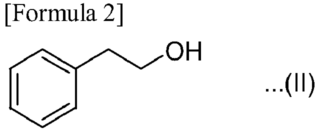

The "phenol derivative" to be comprised in the reagent of the present invention is not particularly limited as long as it is a compound having a phenol structure. The phenol derivative can be selected, for example, from phenol derivatives having a substituent such as a methyl group, an ethyl group, a propyl group, an isopropyl group, a butyl group, an isobutyl group, a tert-butyl group, a methyl carboxylate group, an ethyl carboxylate group, a propyl carboxylate group, and a butyl carboxylate group. Particularly isopropyl methylphenol (Formula III) and propylparaben (Formula IV) can be preferably used.

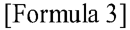

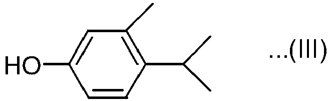

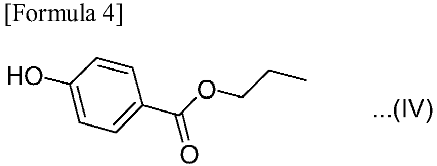

The "polyhydric fatty alcohol" to be comprised in the reagent of the present invention is not particularly limited as long as it is a fatty alcohol having two or more hydroxyl groups. The polyhydric fatty alcohol can be selected, for example, from ethylene glycol, glycerin, propanediol (propylene glycol), butanediol (butylene glycol), butanetriol, pentanediol, pentanetriol, hexanediol, hexanetriol, heptanediol, heptanetriol, octanediol, octanetriol, cyclopentanediol, cyclopentanetriol, methyl pentanediol, cyclohexanediol, cyclohexanetriol, methylcyclohexanediol, cycloheptanediol, cycloheptanetriol, cyclooxanediol, cyclooxanetriol, and derivatives of these. Particularly, glycerin, 1,3-butylene glycol (Formula V) can be preferably used.

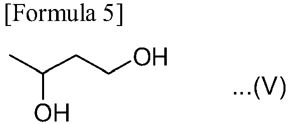

The reagent of the present invention preferably comproses an aromatic alcohol. In particular, phenoxyethanol is more preferably comprised since it can stabilize a specimen at a low concentration for a long time.

The content of aromatic alcohol or the like to be comprised in the reagent of the present invention in the case where a urine specimen is applied, is preferably 0.5 to 100 mg, and particularly preferably 1.0 to 50 mg per mL of the urine specimen. When phenoxyethanol is comprised, the content thereof in the case where a urine specimen is applied, is preferably 1.0 to 50 mg, and particularly preferably 7.5 to 20 mg per mL of the urine specimen.

The urine specimen preserved with the reagent of the present invention is subjected to measurement of urine components. Before measurement, it is necessary to extract the components to be measured from a filter paper with a liquid medium. More specifically, for example, a method can be used in which a filter paper strip is dipped in a liquid medium and shaken for a predetermined time, and then, the supernatant is collected. However, the extraction method is not limited to this. As the liquid medium, e.g., water, saline, or PBS can be preferably used.

The urine components (items) to be measured may be those usually measured in urinalysis. Examples of the urine components include, but are not limited to, sugar, protein, occult blood, ketone bodies, urobilinogen, bilirubin, albumin, creatine, creatinine, inulin, urea nitrogen (UN), cystatin C, uric acid (UA), amino acid, hydroxyproline, citric acid, oxalic acid, nitrite, phosphate, amylase, lysozyme, N-acetylglucosaminidase, sodium, potassium, chlorine, magnesium, calcium, inorganic phosphorus, iron, copper, zinc, manganese, lead, chromium, cadmium, mercury, acetone, methanol, 8-hydroxydeoxyguanosine (8-OHdG), isoprostane, pentosidine, interleukin-6, coproporphyrin, uroporphyrin, porphobilinogen, δ-aminolevulinic acid, N-acetyl β-D-glucosaminidase, deoxypyridinoline, estrone, estradiol, equol, isoflavones, indoxyl sulfuric acid, cortisol, aldosterone, catecholamine, metanephrine fraction, human chorionic gonadotropin (hCG), pregnanediol, pregnantriol, transferrin, type IV collagen, cross-linked N-telopeptide of type I collagen, cross-linked C-telopeptide of type II collagen, C-peptide, L-form fatty acid-binding protein (L-FABP), fatty acid, α1-microglobulin, β2-microglobulin, myoglobin, fibrin/fibrinogen degradation product, nicotine, cotinine, cAMP, folic acid, and vitamins. Of them, the reagent of the present invention is particularly useful for analysis of urine components such as 8-OHdG, vitamins (for example, vitamin B1, vitamin B2), and metal ions (for example, magnesium) whose measurement values are significantly affected by oxidation of a specimen.

8-OHdG is a molecule produced by modifying a carbon atom at the 8-position of a guanidine base with OH when DNA is oxidatively damaged by active oxygen, as shown in Formula VI. The 8-OHdG in DNA is excised out from DNA by the action of a DNA repair enzyme and discharged from cells into urine. Thus, 8-OHdG is known as a biomarker reflecting *in-vivo* oxidative stress.

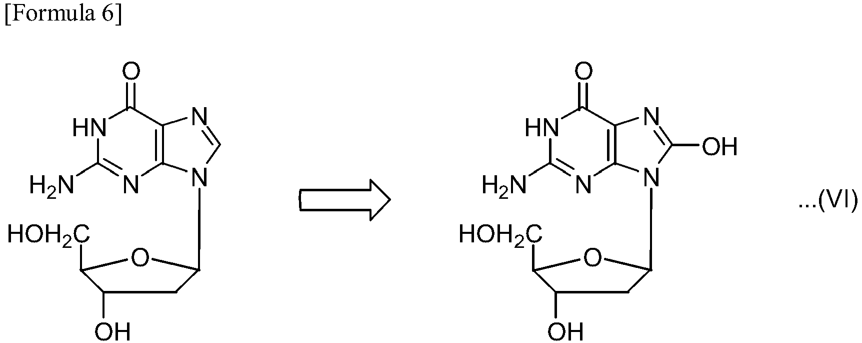

However, particularly when a urine specimen is put in an environment exposed to oxygen for a long time, like a specimen subjected to urinalysis by mail, i.e., a urine-impregnated filter paper, unoxidized guanidine is oxidized, causing a risk of falsely increasing the measurement value of 8-OHdG in some cases. As described above, in the cases of items presumed to be likely to be affected by exposure to oxygen, it is useful to use a reagent that can avoid or reduce the effect of oxygen.

Although a means for measuring 8-OHdG is not particularly limited, 8-OHdG can be measured, for example, by immunoassay using an anti-8-OHdG monoclonal antibody. For measurement of a low-molecular substance such as 8-OHdG, preferably a competition technique can be used in accordance with the principle, for example, shown in Figure 1. On the surface of, e.g., a micro well plate or magnetic beads (in the figure, a micro well plate 11), an antigen 12 (8-OHdG) is immobilized (A). Subsequently, a sample comprising an antigen 13 (8-OHdG) is added (B). To this, a labeled anti-8-OHdG antibody 14 is added to induce an antigen-antibody reaction (C). Molecules not bound are removed by washing (D). If the label is an enzyme, for example, a substrate for the enzyme is added and a signal derived from the label is detected. The larger the amount of 8-OHdG (antigen) in a sample, the larger the amount of the labeled antibody bound to the antigen in the sample and removed by washing. Because of this, low-intensity signal is resulted. In contrast, if the amount of the antigen in a sample is low, high-intensity signal is resulted. The amount thereof can be determined based on a calibration curve, which is prepared by using a plurality of 8-OHdG solutions having known different concentrations as standard solutions and signals obtained in the same manner as in the sample. The amount of 8-OHdG in the sample can be calculated by comparing the signal emitted from a sample with the calibration curve.

The method for labeling an antibody is not particularly limited as long as it can be used for immunoassay. Any known methods such as labeling with an enzyme (e.g., horseradish peroxidase (HRP), alkaline phosphatase (ALP)), fluorescent labeling, and isotope labeling, can be used. Labeling with an enzyme can be preferably used since, e.g., a special facility is not required.

As a method for measuring vitamins B1 and B2, any known method that is commonly used can be used. As a method for measuring vitamin B1, for example, measurement by high performance liquid chromatography (HPLC) and measurement by LC/MS/MS are known. As a method for measuring vitamin B2, for example, measurement by HPLC and measurement by LC/MS/MS are known.

As a method for measuring magnesium, any known method that is commonly used (for example, colorimetric method (xylidyl blue method) and an enzymatic method) can be used. A method for measuring other metal ions, any known method that is commonly used can be used.

It was demonstrated, in Examples, that time-dependent changes of measurement values of 8-OHdG in a urine-impregnated filter paper allowed to stand still at 25°C and 37°C are reduced by use of the reagent of the present invention, compared to the case where a conventional filter paper is used.

### <2. Kit>

The kit of the present invention comprises the reagent disclosed in the section <1. Reagent>. As described above, the "reagent" includes not only a compound but also a filter paper comprising the compound. Detailed conditions for the kit of the present invention are the same as those disclosed in the section <1. Reagent>, unless otherwise specified.

The kit of the present invention may comprise not only the reagent but also instructions for explaining how to prepare a urine specimen to a subject who uses urinalysis by mail. In addition, the kit may comprise, if necessary, e.g., a urine collection container such as a urine cup, a plastic bag with zipper for preserving a urine specimen prepared, a mailing envelope, and a form for filling out information about a subject.

Alternatively, the kit of the present invention may comprise not only a reagent for preserving a urine specimen but also a reagent for measuring a desired urine component. The desired urine component herein is not particularly limited as long as it is ordinarily measured by urinalysis. The kit of the present invention is suitable for measuring a component likely affected by oxidation, for example, 8-OHdG. Accordingly, the kit preferably comprises a reagent for measuring 8-OHdG. The type of a reagent for measuring 8-OHdG and measurement conditions are as disclosed in the section <1. Reagent>, unless otherwise specified.

### <3. Method>

The method of the present invention is a method for preserving a urine specimen, comprising a step of bringing the urine specimen into contact with a filter paper comprising an aromatic alcohol, a phenol derivative, or a polyhydric fatty alcohol. Detailed conditions for the method of the present invention are the same as those disclosed in the section <1. Reagent>, unless otherwise specified.

In the method of the present invention, it is required for a urine-impregnated filter paper to comprise an aromatic alcohol or the like. More specifically, a filter paper is impregnated with an aromatic alcohol or the like, in advance, and if necessary, dried, and then, urine is brought into contact with the filter paper (e.g., by dripping or impregnating). In this manner, the resultant urine-impregnated filter paper can comprise an aromatic alcohol or the like.

The amount of aromatic alcohol or the like to be comprised in a filter paper is preferably 0.5 to 100 mg and particularly preferably 1.0 to 50 mg per mL of a urine specimen.

The method of the present invention may comprise a step of extracting a desired urine component from a preserved urine specimen. For a urine-impregnated filter paper, a step of extracting a component to be measured from the filter paper with a liquid medium is required. The step of extracting a urine component may be a step of, for example, dipping a filter paper strip in a liquid medium, shaking it for a predetermined time, and then, collecting the supernatant. However, the step is not limited to this. The liquid medium that can be used is preferably, e.g., water, saline, and PBS.

The method of the present invention may comprise a step of measuring a desired urine component in a sample extracted from a urine-impregnated filter paper. The desired urine component herein is not particularly limited as long as it is ordinarily measured by urinalysis. The method of the present invention is suitable for measuring a component likely affected by oxidation, for example, 8-OHdG. Accordingly, the method of the present invention preferably comprises a step of measuring 8-OHdG. A procedure, principle, and others for measuring 8-OHdG are the same as those disclosed in the section <1. Reagent>, unless otherwise specified.

### Examples

Now, the present invention will be more specifically described by way of Examples but the present invention is not limited to these Examples.

### <Example 1: Preservation stability test of urine-impregnated filter paper using filter paper comprising phenoxyethanol>

### (1) Preparation of sample

A filter paper (cellulose, thickness of 1.4 mm) was cut into pieces to prepare filter paper strips of 7 mm × 14 mm. The filter paper strips were each put in 1.5 mL polypropylene tubes and 45 µL of a 2.0% phenoxyethanol/ethanol solution was added. The strips were dried with the lid open by a vacuum dryer. To the filter paper dried, 90 µL of a urine specimen comprising 200 ng/mL of 8-OHdG was added (final concentration of phenoxyethanol was 1.0%/urine). The tubes were incubated at 25°C and 37°C for 0, 7, 14, and 21 days. After completion of incubation for a predetermined period, the specimens were stored at -20°C. After incubation of all specimens was completed, the temperature was returned to normal temperature. The specimens were dried by a vacuum dryer to prepare urine-impregnated filter paper specimens. To the tubes comprising urine-impregnated filter paper specimens, 900 µL of PBS was added and the resultant was stirred at room temperature for 1 hour while shaking. After completion of stirring, individual supernatants were collected to obtain samples for measurement. The test was carried out by using urine samples taken from two human subjects (subjects A and B).

### (2) Measurement of 8-OHdG

The measurement principle of 8-OHdG is schematically shown in the above section and Figure 1. An 8-OHdG-BSA complex was diluted with PBS. 50 µL of the diluted solution was dispensed in each of the wells of a 96-well microplate and the microplate was shaken at room temperature for 1 hour. After the microplate was washed with a 0.01% Tween 20-PBS (TPBS) four times, 250 µL of 1% BSA/PBS was added. The microplate was incubated at room temperature for 1 hour. After washing with TPBS four times, the individual samples prepared in (1), a positive control, and an 8-OHdG standard solution having a known concentration were each added in a volume of 50 µL. 50 µL of a solution of a HRP-labeled anti-8-OHdG antibody was dispensed. The microplate was shaken for 10 minutes and thereafter incubated at room temperature for 50 minutes. After the microplate was washed with TPBS four times, 100 µL of tetramethylbenzidine (TMB) was dispensed. The microplate was allowed to stand still for 10 minutes to produce a color. The color-producing reaction was terminated by dispensing 50 µL of a 1 M phosphoric acid. Absorbance was measured at 450 nm/570 nm.

### (3) Results

The measurement results of individual samples are shown in Table 1 and Table 2. The numerical values in the tables were computationally obtained by regarding the measurement value of 8-OHdG in a specimen measured in the same conditions on Day 0 as 100. Table 1 shows the measurement results of urine-impregnated filter paper incubated at 25°C. Table 2 shows the measurement results of urine-impregnated filter paper incubated at 37°C. The measurement values underlined represent the numerical values beyond 10% as high as and as low as the value of Day 0. It was demonstrated that stable measurement values are obtained in urine-impregnated filter papers comprising phenoxyethanol in both conditions of 25°C and 37°C.

**[Table 1]**

| 25°C | Phenoxyethanol/Day | Day0 | Day7 | Day14 | Day21 |
|---|---|---|---|---|---|
| Subject A | Not added | 100 | 110.7 | 94.8 | 103.3 |
| | 1.0% | 100 | 95.0 | 99.3 | 109.9 |
| Subject B | Not added | 100 | 105.3 | 119.8 | 104.5 |
| | 1.0% | 100 | 98.0 | 100.4 | 109.2 |

**[Table 2]**

| 37°C | Phenoxyethanol/Day | Day0 | Day7 | Day14 | Day21 |
|---|---|---|---|---|---|
| Subject A | Not added | 100 | 89.4 | 86.5 | 71.1 |
| | 1.0% | 100 | 105.1 | 100.7 | 106.6 |
| Subject B | Not added | 100 | 101.4 | 95.9 | 70.9 |
| | 1.0% | 100 | 105.8 | 99.0 | 102.1 |

### <Example 2: Preservation stability test (8-OHdG) of urine-impregnated filter paper using filter paper comprising various components>

Urine-impregnated filter papers were prepared in the same manner as in Example 1 except that each of the solutions was added in different concentrations as shown in Table 3 in place of the 1.0% phenoxyethanol/ethanol solution. The tubes comprising different urine-impregnated filter papers were incubated at 25°C and 37°C for 0, 7, and 14 days. The specimens after completion of incubation for a predetermined period were stored at -20°C. After incubation of all specimens was completed, the temperature was returned to normal temperature. The specimens were dried by a vacuum dryer to prepare urine-impregnated filter paper specimens. To the tubes comprising urine-impregnated filter paper specimens, 900 µL of PBS was added and the resultant was stirred at room temperature for 1 hour while shaking. After completion of stirring, individual supernatants were collected to obtain samples for measurement. The test was carried out by using a urine sample taken from a single human subject. Measurement of 8-OHdG was performed in the same manner as in Example 1.

**[Table 3]**

| | Concentration (%) | Final urine concentration (%) | Solvent |
|---|---|---|---|
| Phenoxyethanol | 0.2 | 0.1 | Ethanol |
| | 1.0 | 0.5 | |
| | 2.0 | 1.0 | |
| | 10 | 5.0 | |
| | 20 | 10 | |
| Phenethyl alcohol | 0.2 | 0.1 | Ethanol |
| | 1.0 | 0.5 | |
| | 2.0 | 1.0 | |
| | 10 | 5.0 | |
| | 20 | 10 | |
| Isopropyl methyl phenol | 0.2 | 0.1 | Methanol |
| | 1.0 | 0.5 | |
| | 2.0 | 1.0 | |
| | 10 | 5.0 | |
| | 20 | 10 | |
| Propylparaben | 0.2 | 0.1 | Methanol |
| | 1.0 | 0.5 | |
| | 2.0 | 1.0 | |
| | 10 | 5.0 | |
| | 20 | 10 | |
| 1,3-Butylene glycol | 0.2 | 0.1 | Ultrapure water |
| | 1.0 | 0.5 | |
| | 2.0 | 1.0 | |
| | 10 | 5.0 | |
| | 20 | 10 | |

The measurement results of 8-OHdG in urine-impregnated filter papers comprising an aromatic alcohol, a phenol derivative, and a polyhydric fatty alcohol are shown in Table 4, Table 5, and Table 6, respectively. The numerical values in the tables were computationally obtained by regarding the measurement value of 8-OHdG in a specimen measured in the same conditions on Day 0 as 100. The measurement values underlined represent the numerical values beyond 10% as high as and as low as the value of Day 0. The stability of a urine-impregnated filter paper comprising phenoxyethanol particularly at a final concentration of 1.0% was excellent compared to a urine-impregnated filter paper not comprising phenoxyethanol. The stability of a urine-impregnated filter paper comprising phenethyl alcohol was excellent at final concentrations of 1.0% and 5.0%. Focusing limitedly on a preservation period of 7 days, the stability of a urine-impregnated filter paper comprising phenethyl alcohol was excellent at any concentration compared to the urine-impregnated filter paper not comprising phenethyl alcohol. The stability of a urine-impregnated filter paper comprising isopropyl methylphenol was excellent at a final concentration of 5.0%. The stability of a urine-impregnated filter paper comprising propylparaben was excellent at a final concentration of 0.5 to 5.0%. The stability of a urine-impregnated filter paper comprising butylene glycol was found to be high at any concentration.

The above results demonstrated that variation in measurement value of 8-OHdG by preservation of specimens can be suppressed in urine-impregnated filter papers comprising phenoxyethanol, phenethyl alcohol, isopropyl methylphenol, propylparaben, and butylene glycol.

**[Table 4]**

| | | Day0 | Day7 | Day14 |
|---|---|---|---|---|
| Not added | 25°C | 100 | 113.66 | 113.54 |
| | 37°C | 100 | 110.13 | 110.20 |

| Phenoxyethanol | | | | |
|---|---|---|---|---|
| 0.1% | 25°C | 100 | 108.82 | 103.86 |
| | 37°C | 100 | 105.89 | 110.81 |
| 0.5% | 25°C | 100 | 106.08 | 102.46 |
| | 37°C | 100 | 112.01 | 108.73 |
| 1.0% | 25°C | 100 | 96.60 | 98.26 |
| | 37°C | 100 | 94.71 | 104.74 |
| 5.0% | 25°C | 100 | 105.07 | 114.49 |
| | 37°C | 100 | 124.52 | 121.58 |
| 10% | 25°C | 100 | 84.77 | 79.82 |
| | 37°C | 100 | 75.42 | 76.77 |

| Phenethyl alcohol | | | | |
|---|---|---|---|---|
| 0.1% | 25°C | 100 | 109.91 | 108.70 |
| | 37°C | 100 | 106.66 | 107.01 |
| 0.5% | 25°C | 100 | 99.41 | 108.91 |
| | 37°C | 100 | 106.60 | 112.73 |
| 1.0% | 25°C | 100 | 98.27 | 94.82 |
| | 37°C | 100 | 101.21 | 94.73 |
| 5.0% | 25°C | 100 | 99.76 | 106.72 |
| | 37°C | 100 | 103.91 | 104.06 |
| 10% | 25°C | 100 | 99.90 | 97.95 |
| | 37°C | 100 | 100.48 | 116.57 |

**[Table 5]**

| | | Day0 | Day7 | Day14 |
|---|---|---|---|---|
| Not added | 25°C | 100 | 113.66 | 113.54 |
| | 37°C | 100 | 110.13 | 110.20 |

| Isopropyl methylphenol | | | | |
|---|---|---|---|---|
| 0.1% | 25°C | 100 | 117.79 | 109.90 |
| | 37°C | 100 | 135.21 | 112.44 |
| 0.5% | 25°C | 100 | 116.80 | 101.84 |
| | 37°C | 100 | 111.06 | 109.17 |
| 1.0% | 25°C | 100 | 111.03 | 99.92 |
| | 37°C | 100 | 97.32 | 105.46 |
| 5.0% | 25°C | 100 | 96.78 | 95.02 |
| | 37°C | 100 | 106.65 | 114.36 |
| 10% | 25°C | 100 | 89.23 | 105.31 |
| | 37°C | 100 | 87.26 | 93.08 |

| Propylparaben | | | | |
|---|---|---|---|---|
| 0.1% | 25°C | 100 | 117.46 | 106.92 |
| | 37°C | 100 | 109.30 | 107.89 |
| 0.5% | 25°C | 100 | 102.54 | 97.29 |
| | 37°C | 100 | 103.21 | 103.04 |
| 1.0% | 25°C | 100 | 100.88 | 85.29 |
| | 37°C | 100 | 98.85 | 96.14 |
| 5.0% | 25°C | 100 | 94.22 | 104.33 |
| | 37°C | 100 | 106.65 | 105.67 |
| 10% | 25°C | 100 | 98.14 | 110.60 |
| | 37°C | 100 | 106.23 | 98.58 |

**[Table 6]**

| | | Day0 | Day7 | Day14 |
|---|---|---|---|---|
| Not added | 25°C | 100 | 113.66 | 113.54 |
| | 37°C | 100 | 110.13 | 110.20 |

| Butylene glycol | | | | |
|---|---|---|---|---|
| 0.1% | 25°C | 100 | 102.79 | 103.57 |
| | 37°C | 100 | 103.54 | 100.97 |
| 0.5% | 25°C | 100 | 97.02 | 97.29 |
| | 37°C | 100 | 108.61 | 101.16 |
| 1.0% | 25°C | 100 | 98.43 | 93.34 |
| | 37°C | 100 | 95.15 | 92.24 |
| 5.0% | 25°C | 100 | 97.83 | 97.13 |
| | 37°C | 100 | 104.02 | 101.71 |
| 10% | 25°C | 100 | 101.57 | 101.57 |
| | 37°C | 100 | 105.83 | 95.76 |

### <Example 3: Test on stability of measurement values of analytes in urine-impregnated filter paper>

Whether measurement values of 8-OHdG, vitamin B1, vitamin B2, and magnesium in a urine-impregnated filter paper specimen are stably obtained was examined. A urine-impregnated filter paper was prepared in the same manner as in Example 1 except that urine not comprising 8-OHdG was used. As a control, a urine-impregnated filter paper was prepared using a filter paper not comprising phenoxyethanol. Tubes comprising different urine-impregnated filter papers were incubated at 25°C and 37°C for a predetermined period within 0 to 21 days. After completion of incubation for a predetermined period, specimens were stored at -20°C. After incubation of all specimens was completed, the temperature was returned to normal temperature. The specimens were dried by a vacuum dryer to prepare urine-impregnated filter paper specimens. To the tubes comprising urine-impregnated filter paper specimens, 900 µL of PBS was added and the resultant was stirred at room temperature for 1 hour while shaking. After completion of stirring, individual supernatants were collected to obtain samples for measurement.

Measurement of 8-OHdG of urine-impregnated filter paper specimens was performed (n = 10) in the same manner as in Example 1. Vitamin B1 and vitamin B2 in each urine-impregnated filter paper specimen were measured by HPLC (n = 3 for each). Magnesium in each urine-impregnated filter paper specimen was measured by an ordinary enzymatic method (n = 8).

The relationship between analytes and incubation time of urine-impregnated filter papers are shown in Figures 2 to 5. Figure 2 discloses graphs showing the relationship between the variation rate of 8-OHdG measurement value and the incubation time. Figure 2A shows the variation rate of the measurement value of urine-impregnated filter paper specimen not comprising phenoxyethanol and Figure 2B shows the variation rate of the measurement value of the specimen comprising phenoxyethanol. Figure 3 discloses graphs showing the relationship between the variation rate of vitamin B1 measurement value and the incubation time. Figure 3A shows the variation rate of the measurement value of urine-impregnated filter paper specimen not comprising phenoxyethanol and Figure 3B shows the variation rate of the measurement value of the specimen comprising phenoxyethanol. Figure 4 discloses graphs showing the relationship between the variation rate of vitamin B2 measurement value and the incubation time. Figure 4A shows the variation rate of the measurement value of urine-impregnated filter paper specimen not comprising phenoxyethanol and Figure 4B shows the variation rate of the measurement value of the specimen comprising phenoxyethanol. Figure 5 discloses graphs showing the relationship between the variation rate in magnesium measurement value and the incubation time. Figure 5A shows the variation rate of the measurement value of urine-impregnated filter paper specimen not comprising phenoxyethanol and Figure 5B shows the variation rate of the measurement value of the specimen comprising phenoxyethanol.

It was confirmed that no significant variation is observed in 8OHdG measurement value by addition of phenoxyethanol even if the filter paper is allowed to leave for up to 21 days; that measurement values of vitamin B1 and vitamin B2 significantly vary during a short period of time in the absence of phenoxyethanol but are relatively stable by addition of phenoxyethanol from Day 7 to 14; and that no significant variation is observed in magnesium measurement value by addition of phenoxyethanol even if the filter paper is allowed to leave for up to 21 days.

### <Example 4: Variation in creatinine measurement value by the degree of dryness of urine-impregnated filter paper>

Whether a measurement value of creatinine in a urine-impregnated filter paper specimen is stably obtained was examined. A urine-impregnated filter paper was prepared in the same manner as in Example 3. As a control, a urine-impregnated filter paper was prepared using a filter paper not comprising phenoxyethanol (ordinary specimen). Tubes comprising different urine-impregnated filter papers were incubated at 25°C and 37°C for a predetermined period within 0 to 21 days. In this case, it was confirmed that all filter papers were dry in appearance after the elapse of 7 days. In the meantime, to a filter paper strip comprising/not comprising phenoxyethanol, 90 µL of a urine specimen was added. The filter paper strip was then dried in air at room temperature for 6 hours and subsequently allowed to stand still under the conditions of 25°C and 37°C for a predetermined time in open air (dry specimen). After completion of incubation for a predetermined period, specimens were stored at -20°C. After incubation of all specimens was completed, the temperature was returned to normal temperature. The specimens were dried by a vacuum dryer to prepare urine-impregnated filter paper specimens. To the tubes comprising urine-impregnated filter paper specimens, 900 µL of PBS was added and the resultant was stirred at room temperature for 1 hour while shaking. After completion of stirring, individual supernatants were collected to obtain samples for measurement. Creatinine in each urine-impregnated filter paper specimen was measured by an ordinary enzymatic method (n = 7).

Figure 6 discloses graphs showing the relationship between the variation rate of the creatinine measurement value in an ordinary urine-impregnated filter paper specimen and the incubation time. Figure 6A shows the variation rate of the measurement value of urine-impregnated filter paper specimen not comprising phenoxyethanol and Figure 6B shows the variation rate of the measurement value of the specimen comprising phenoxyethanol. The effect of phenoxyethanol on the creatinine measurement value was not virtually observed. The creatinine measurement value gradually decreased in a high temperature state of 37°C and tended to significantly decrease over 20% particularly after 7 days, whereas the creatinine measurement value was relatively stable under a condition of 25°C. From these, it was found that in order to accurately measure a urine-impregnated filter paper specimen, it is desired to appropriately control the temperature or treat a specimen in an earlier stage.

Figure 7 discloses graphs showing the relationship between the variation rate of the creatinine measurement value in a dried urine-impregnated filter paper specimen and the incubation time. Figure 7A shows the variation rate of the measurement value of urine-impregnated filter paper specimen not comprising phenoxyethanol and Figure 7B shows the variation rate of the measurement value of the specimen comprising phenoxyethanol. In a dried urine-impregnated filter paper, downward tendency of the creatinine measurement value was not observed. Note that, when 8-OHdG, vitamins B1 and B2, and magnesium for a dried urine-impregnated filter paper specimen comprising 1% phenoxyethanol were measured in the same manner, an average measurement value of a plurality of specimens (n = 4 to 8) was confirmed to be stabilized in the same level as in ordinary specimens (data not shown). It is difficult to allow a urine-impregnated filter paper to stand still in an open container from a clinical point of view but it was demonstrated to be desirable to dry a urine-impregnated filter paper in an earlier stage by use of, e.g., desiccant, in the case where a specimen is required to be stored for a long period of time in the condition where temperature control is difficult.

### <Example 5: Dry state of urine-impregnated filter paper by preservation means>

To one of the ends of a band-like plastic thin strip having a width of 7 mm × length of 85 mm, in a longitudinal direction, a filter paper (cellulose, thickness of 1.4 mm) having a width of 7 mm × length of 14 mm was attached to prepare a filter-paper stick. The portion of the plastic thin piece having no filter paper was used as a handheld part. A urine specimen was put in a urine cup and a filter paper portion of the filter-paper stick was dipped in the urine specimen to impregnate the filter paper with urine. When the filter-paper stick was pulled up from the urine specimen, the filter paper portion was pressed onto the urine-cup wall to remove excessive liquid. A plastic bag with zipper (Horiaki Co., Ltd., product name "Wrap-in," PE-4) comprising a desiccant (manufactured by Toyota Kako Co., Ltd., 10 g of silica gel) and a plastic bag with zipper not comprising a desiccant were prepared. A filter-paper stick impregnated with urine was put in each of the plastic bags comprising a desiccant and no desiccant. The plastic bags were allowed to stand still at room temperature for 6 days. A change in weight of the filter-paper sticks was monitored during the time period. Similarly, these plastic bags were put in a desiccator and allowed to stand still for 6 days. A change in weight of the filter-paper sticks was monitored. The room temperature during the monitoring period was 27°C and the relative humidity thereof was 33 to 55%. The temperature in a desiccator was 27°C and the relative humidity thereof was 24 to 33%. The numbers of plastic bags and filter-paper stick urine specimens prepared were 3 for each condition.

Table 7 shows a change in weight of filter-paper sticks in plastic bags comprising a desiccant and no desiccant allowed to stand still at room temperature. Based on the difference in weight of a filter-paper stick between before and immediately after impregnation with urine regarded as 100%, the time until the difference in weight of the filter-paper stick impregnated with urine from that before impregnation with urine became 10% or less was defined as "drying time." The required time was measured. In the condition comprising a desiccant, the drying time was 19.5 to 24 hours. On the other hand, in the condition comprising no desiccant, the time required for the stick to reach the same state was 115.5 hours. Table 8 shows a change in weight of filter papers in plastic bags comprising a desiccant and no desiccant allowed to stand still in a desiccator. In the condition comprising a desiccant, the drying time was 19.5 to 24 hours. On the other hand, in the condition comprising no desiccant, the time required for the stick to reach the same state was 115.5 to 125 hours. Even in a low-humidity desiccator, the drying time was not particularly reduced. From the above, it was demonstrated that when a urine specimen is put in a plastic bag and allowed to leave at room temperature, the urine-impregnated filter paper is dried in about 1 day in a plastic bag comprising a desiccant and about 5 to 6 days in a plastic bag comprising no desiccant.

**[Table 7]**

| Weight (mg) | Presence of desiccant | | | Absence of desiccant | | |
|---|---|---|---|---|---|---|
| | 1 | 2 | 3 | 1 | 2 | 3 |
| Before impregnation with urine | 291.09 | 287.02 | 291.03 | 292.28 | 286.51 | 293.30 |
| After impregnation with urine | 428.55 | 421.41 | 432.04 | 437.51 | 420.14 | 434.61 |
| 4.5 hours later | 405.48 | 350.82 | 405.33 | 428.55 | 410.12 | 423.96 |
| 19.5 hours later | 302.38 | 291.71 | 313.69 | 413.60 | 389.55 | 405.94 |
| 24 hours later | 298.52 | 291.84 | 298.50 | 406.92 | 379.41 | 398.30 |
| 28 hours later | 298.24 | 291.32 | 297.54 | 400.00 | 372.36 | 389.65 |
| 48 hours later | 298.14 | 291.49 | 297.42 | 360.64 | 343.72 | 352.42 |
| 78 hours later | 298.14 | 291.71 | 297.51 | 328.44 | 307.64 | 319.17 |
| 115.5 hours later | 297.29 | 291.65 | 297.11 | 304.19 | 292.43 | 300.98 |
| 120.5 hours later | 297.86 | 291.34 | 297.11 | 302.35 | 292.13 | 300.65 |
| 125 hours later | 297.95 | 291.31 | 297.12 | 301.62 | 292.02 | 300.54 |
| 140 hours later | 298.00 | 291.32 | 297.12 | 301.43 | 292.20 | 300.65 |

**[Table 8]**

| Weight (mg) | Presence of desiccant | | | Absence of desiccant | | |
|---|---|---|---|---|---|---|
| | 1 | 2 | 3 | 1 | 2 | 3 |
| Before impregnation with urine | 288.75 | 290.72 | 284.31 | 285.76 | 288.43 | 289.76 |
| After impregnation with urine | 427.88 | 435.90 | 427.15 | 423.52 | 430.35 | 433.33 |
| 4.5 hours later | 383.93 | 398.40 | 373.34 | 412.56 | 415.31 | 422.22 |
| 19.5 hours later | 296.83 | 309.88 | 289.37 | 393.94 | 401.47 | 404.60 |
| 24 hours later | 296.43 | 297.75 | 289.15 | 388.80 | 395.34 | 395.69 |
| 28 hours later | 296.39 | 297.30 | 289.22 | 382.92 | 389.80 | 388.42 |
| 48 hours later | 296.29 | 297.12 | 288.93 | 352.24 | 369.40 | 363.70 |
| 78 hours later | 296.38 | 297.69 | 288.97 | 320.76 | 344.73 | 330.58 |
| 115.5 hours later | 296.25 | 297.91 | 288.42 | 296.70 | 309.55 | 304.80 |
| 121.5 hours later | 296.85 | 297.88 | 288.70 | 295.35 | 304.42 | 300.83 |
| 125 hours later | 296.00 | 297.88 | 288.75 | 294.99 | 301.23 | 299.34 |
| 140 hours later | 296.10 | 297.82 | 288.89 | 294.38 | 295.35 | 298.05 |

All publications, patents, and patent applications cited herein are incorporated in their entireties by reference.

## Claims

1. A reagent for preserving a urine specimen, comprising a filter paper, wherein the filter paper comprises an aromatic alcohol, a phenol derivative, or a polyhydric fatty alcohol.

2. The reagent according to claim 1, wherein the content of the aromatic alcohol, phenol derivative, or polyhydric fatty alcohol in the filter paper is 0.5 to 100 mg per mL of a urine specimen to be applied.

3. The reagent for preserving a urine specimen according to claim 1 or 2, wherein the filter paper comprises phenoxyethanol.

4. The reagent according to claim 3, wherein the content of phenoxyethanol in the filter paper is 1.0 to 50 mg per mL of a urine specimen to be applied.

5. The reagent according to any one of claims 1 to 4, wherein the urine specimen is a specimen for measuring a content of at least one selected from the group consisting of 8-hydroxydeoxyguanosine, vitamins, and metal ions.

6. A kit comprising the reagent according to any one of claims 1 to 5.

7. A method for preserving a urine specimen, comprising a step of contacting the urine specimen with a filter paper comprising an aromatic alcohol, a phenol derivative, or a polyhydric fatty alcohol.

8. The method according to claim 7, wherein the content of the aromatic alcohol, phenol derivative, or polyhydric fatty alcohol in the filter paper is 0.5 to 100 mg per mL of the urine specimen to be applied.
